(19) Europäisches Patentamt
European Patent Office
Office européen des brevets

(11) **EP 4 455 282 A1**

(12) **EUROPEAN PATENT APPLICATION**
published in accordance with Art. 153(4) EPC

(43) Date of publication:
**30.10.2024 Bulletin 2024/44**

(21) Application number: **22911232.1**

(22) Date of filing: **20.12.2022**

(51) International Patent Classification (IPC):
*C12N 15/09* (2006.01)   *C12N 5/10* (2006.01)
*C12N 9/16* (2006.01)

(52) Cooperative Patent Classification (CPC):
**C12N 5/10; C12N 9/16; C12N 15/09**

(86) International application number:
**PCT/JP2022/046917**

(87) International publication number:
**WO 2023/120530 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.12.2021  JP 2021210431**

(71) Applicant: **OSAKA UNIVERSITY**
**Suita-shi**
**Osaka 565-0871 (JP)**

(72) Inventor: **NAKADA, Shinichiro**
**Suita-shi, Osaka 565-0871 (JP)**

(74) Representative: **J A Kemp LLP**
**80 Turnmill Street**
**London EC1M 5QU (GB)**

Remarks:
The complete document including Reference
Table(s) and the Sequence Listing(s) can be
downloaded from the EPO website

(54) **METHOD FOR PRODUCING GENOME-EDITED CELLS UTILIZING HOMOLOGOUS RECOMBINATION**

(57)    The present invention provides a genome editing method utilizing recombination between homologous chromosomes having different bases at the target site, specifically, (1) a method of using cells in which the function of a gene selected from the group consisting of Lig4 gene, PARP1 gene, XRCC1 gene, MSH2 gene, and SMARCAL1 gene is suppressed, or (2) a method of causing a single-strand break at one site in the neighboring DNA region of the target site on the chromosome of the recipient, and causing a single-strand break at one site different from the site corresponding to the site where the single-strand break is caused on the chromosome of the recipient, on the chromosome of the donor.

Fig. 1

**Description**

[Technical Field]

The present invention relates to a method for producing genome-edited cells utilizing homologous recombination, and mainly relates to a method for producing cells in which a heterozygous mutation is substituted with a normal sequence via homologous recombination between homologous chromosomes.

[Background Art]

With the advent of programmable nucleases such as TALENs and the CRISPR-Cas system, genome-editing technology is currently spreading rapidly. A Cas protein forms a complex with a guide RNA, and this complex binds to a target site on the genome that has a sequence complementary to the guide RNA and cleaves both strands of the DNA. TALENs, the previous generation of genome-editing technology, are fusion proteins of a TALE protein that targets DNA and a nuclease (mainly FokI) that cleaves DNA, but like the CRISPR-Cas system, they cause a double-strand break of DNA at the target site on the genome. When donor DNA to be a repair template is introduced extracellularly, this DNA double-strand break can be used to perform gene knock-in by homologous recombination between the genome and the donor DNA. However, when using a double-strand break by a genome editing system, there is a problem that unintended mutations are more likely to occur at the target site than knock-in by a repair template, and genome mutations also occur at DNA sequences (off-targets) with high similarity to the target sequence.

In view of the above, the present inventors have developed methods that can, by using nickase-type Cas9 with one of the two nuclease sites of Cas9 inactivated, induce homologous recombination by single-strand break in DNA to suppress the occurrence of unintended mutations due to non-homologous end joining as compared with the conventional method by double-strand break in DNA. One of them is a genome editing method that utilizes a tandem nick method by using nickases to nick two sites in the target genome and one site in the donor plasmid containing the repair template (NPL 1 and PTL 1). In addition, the present inventors have further advanced this method to also develop an SNGD method that nicks one site in the target genome and nicks one site in the donor plasmid containing the repair template (a combination of single nicks in the target gene and donor plasmid) (PTL 1).

Moreover, as an application of the genome editing method using homologous recombination by a nickase, the present inventors developed a method of using a homologous chromosome originally present in cells as a repair template instead of exogenous donor DNA (PTL 2). Since this method can avoid the problem of random integration of donor DNA used as a repair template into the genome, safety can be improved, especially in medical applications.

[Citation List]

[Patent Literature]

   [PTL 1] Japanese Patent Application Publication No. 2018-11525
   [PTL 2] International Publication No. WO2020/100361

[Non Patent Literature]

[NPL 1] Nakajima K, et al., Genome Res. 28, 223-230, 2018

[Summary of Invention]

[Technical Problem]

The present invention aims to further improve the efficiency and specificity of genome editing in a genome editing method utilizing recombination between homologous chromosomes.

[Solution to Problem]

When a heterozygous mutation or compound heterozygous mutation exists in a chromosome in a cell, the genetic mutation present in one of the homologous chromosomes (referred to as "allele A") does not exist in the other homologous chromosome (referred to as "allele B"). Here, if homologous recombination can be induced between allele A and allele B, when using allele B as the donor and allele A as the recipient, the mutation in allele A can be repaired to the normal sequence, and conversely, when using allele A as the donor and allele B as the recipient, the mutation can be introduced into the normal sequence of allele B.

The method described in PTL 2 is characterized by specifically inducing recombination between homologous chromosomes at the target site by generating nicks at multiple sites in the neighboring DNA region of the nucleotide to be edited on the chromosome to be the recipient, and generating nicks in at least one of the sites corresponding to the site where the nicks are generated in the chromosome of the recipient, on the chromosome to be the donor.

In order to further improve the efficiency and specificity of genome editing in the method described in PTL 2, the present inventors diligently studied and found that suppressing the function of the Lig4 gene, PARP1 gene, XRCC1 gene, MSH2 gene, or SMARCAL1 gene in the cells to be genome-edited can significantly improve the efficiency of genome editing. Furthermore, the present inventors found that, by adopting a nick pattern different from that of PTL 2, in which a nick is generated at one site in the neighboring DNA region of the mutation site in allele A and a nick is generated at a site different from the site where the nick is generated in allele A at one site in allele B, it is possible to significantly suppress the occurrence of unintended long-chain DNA deletions and improve the specificity of genome editing, and thus completed the present invention.

Consequently, the present invention includes the following embodiments.

(1) A method for producing genome-edited cells, including:

introducing a combination of site-specific nickases that causes single-strand breaks in a neighboring DNA region of a specific site in homologous chromosomes having different bases between the homologous chromosomes at the specific site, inducing homologous recombination using one of the homologous chromosomes as a recipient and the other as a donor, and substituting a base of the recipient with a base of the donor at the specific site, wherein

the cells have suppressed function of a gene selected from the group consisting of Lig4 gene, PARP1 gene, XRCC1 gene, MSH2 gene, and SMARCAL1 gene, and

the combination of site-specific nickases causes single-strand breaks at multiple sites in the neighboring DNA region of the specific site on the chromosome of the recipient and causes a single-strand break at one site corresponding to the site where the single-strand breaks are caused on the chromosome of the recipient, on the chromosome of the donor.

(2) The method according to (1), wherein the base of the recipient to be substituted is a mutant base and the base of the donor is a normal base.

(3) The method according to (1) or (2), wherein each of the site-specific nickases is a CRISPR-Cas system.

(4) A kit for use in the method according to any one of (1) to (3), including:

a combination of site-specific nickases that causes single-strand breaks in a neighboring DNA region of a specific site of homologous chromosomes in cells having different bases between the homologous chromosomes at the specific site, wherein

the cells have suppressed function of a gene selected from the group consisting of Lig4 gene, PARP1 gene, XRCC1 gene, MSH2 gene, and SMARCAL1 gene, and

the combination of site-specific nickases causes single-strand breaks at multiple sites in the neighboring DNA region of the specific site on the chromosome of the recipient and causes a single-strand break at one site corresponding to the site where the single-strand breaks are caused on the chromosome of the recipient, on the chromosome of the donor.

(5) A method for producing genome-edited cells, including:

introducing a combination of site-specific nickases that causes single-strand breaks in a neighboring DNA region of a specific site in homologous chromosomes having different bases between the homologous chromosomes at the specific site, inducing homologous recombination using one of the homologous chromosomes as a recipient and the other as a donor, and substituting a base of the recipient with a base of the donor at the specific site, wherein

the combination of site-specific nickases causes a single-strand break at one site in the neighboring DNA region of the specific site on the chromosome of the recipient and causes a single-strand break at a different site from the site corresponding to the site where the single-strand break is caused on the chromosome of the recipient, at one site on the chromosome of the donor.

(6) The method according to (5), wherein the base of the recipient to be substituted is a mutant base and the base of the donor is a normal base.

(7) The method according to (5) or (6), wherein each of the site-specific nickases is a CRISPR-Cas system.

(8) A kit for use in the method according to any one of (5) to (7), including:

a combination of site-specific nickases that causes single-strand breaks in a neighboring DNA region of a specific site of homologous chromosomes in cells having different bases between the homologous chromosomes at the specific site, wherein

the combination of site-specific nickases causes a single-strand break at one site in the neighboring DNA region of the specific site on the chromosome of the recipient and causes a single-strand break at a different site from the site corresponding to the site where the single-strand break is caused on the chromosome of the recipient, at one site on the chromosome of the donor.

[Advantageous Effects of Invention]

According to the present invention, it has become possible to perform genome editing efficiently and specifically by utilizing homologous recombination between homologous chromosomes. Since the present invention does not use exogenous donor DNA for homologous recombination, and the problem of random integration of donor DNA does not occur as well, so that genome editing can be performed with high safety even when applied to gene therapy and the like.

[Brief Description of Drawings]

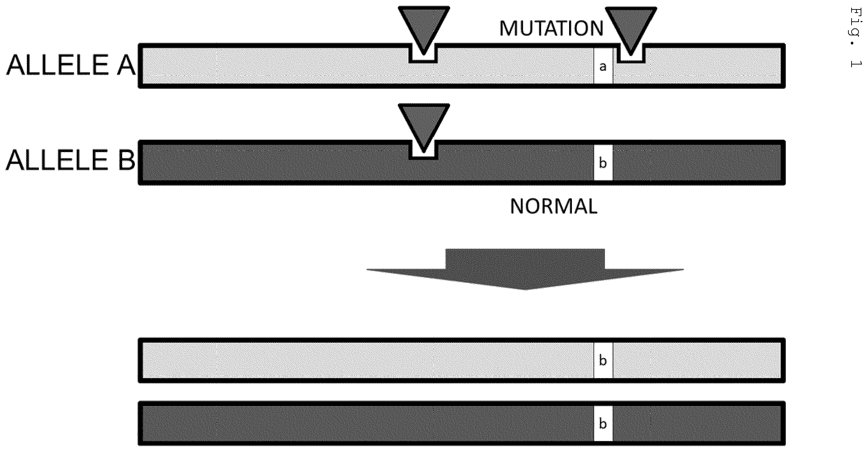

[Fig. 1] Fig. 1 is a diagram showing the principle of the genome editing method of the present invention utilizing homologous recombination between homologous chromosomes (the diagram shows an example of a pattern of causing single-strand breaks at two sites in allele A and one site in allele B).

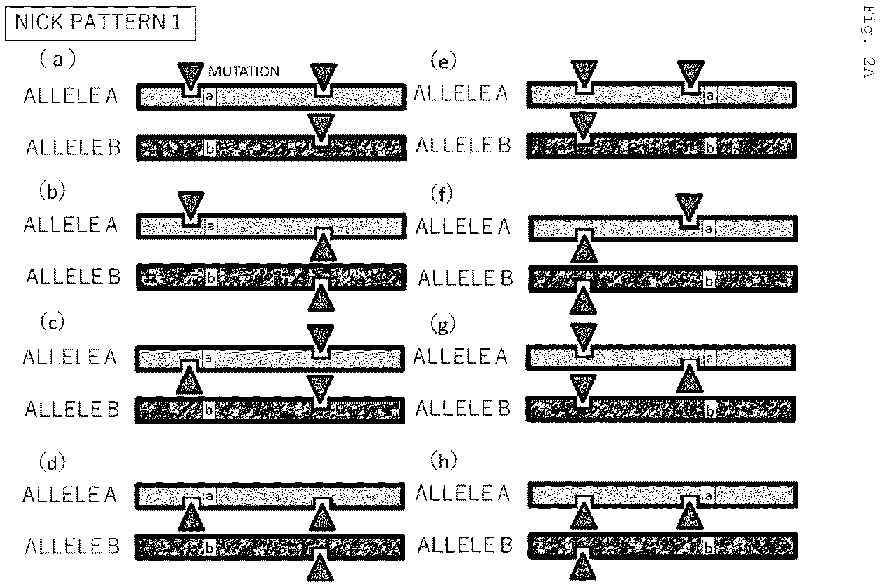

[Fig. 2A] Fig. 2A is a diagram showing an example of a pattern (nick pattern 1) of causing single-strand breaks at two sites in allele A and one site in allele B. The two nicks in allele A: both on the + strand (a, e), one on the + strand and the other on the - strand (b, c, f, g), both on the - strand (d, h). The nick in allele B: on the + strand (a, c, e, g), on the - strand (b, d, f, h).

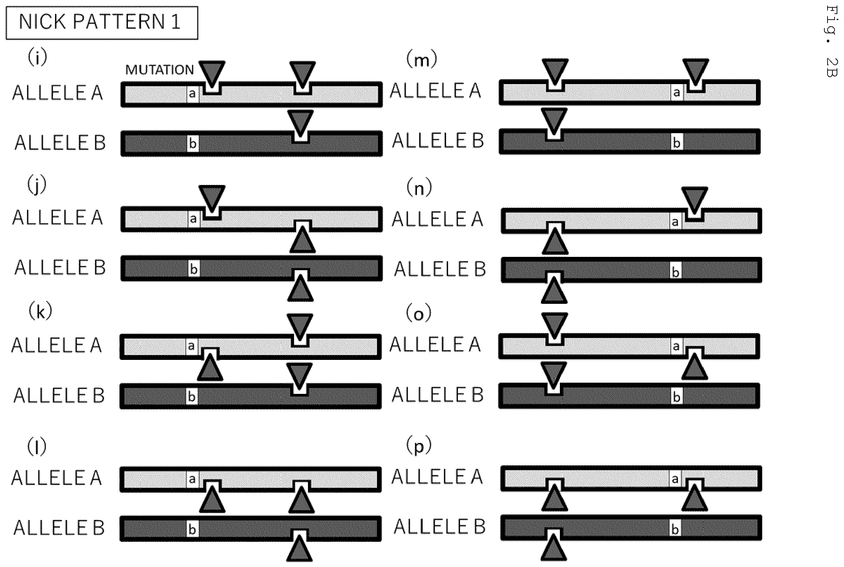

[Fig. 2B] Fig. 2B is a continuation of Fig. 2A. It differs from Fig. 2A in the position of one of the nicks in allele A. The two nicks in allele A: both on the + strand (i, m), one on the + strand and the other on the - strand (j, k, n, o), both on the - strand (l, p). The nick in allele B: on the + strand (i, k, m, o), on the - strand (j, l, n, p).

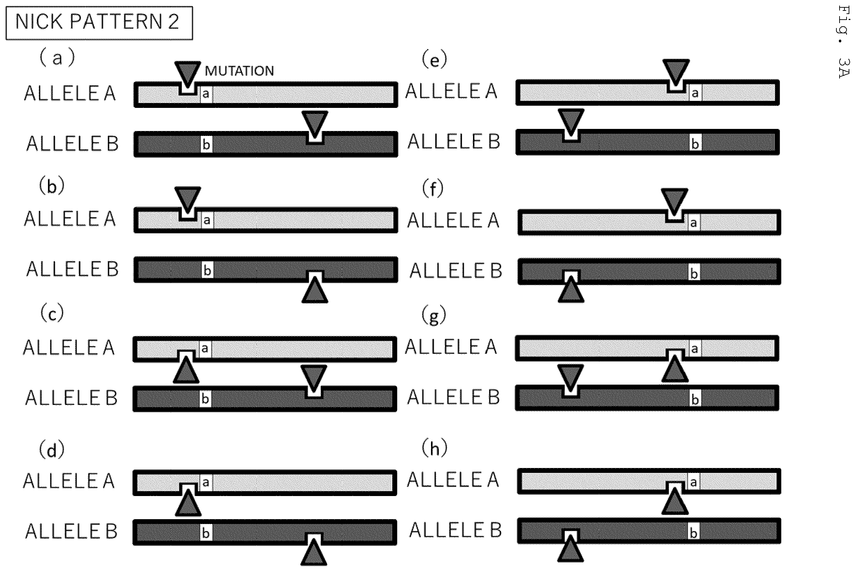

[Fig. 3A] Fig. 3A is a diagram showing an example of a pattern (nick pattern 2) of causing a single-strand break at one site each in allele A and allele B. The nick in allele A: on the + strand (a, b, e, f), on the - strand (c, d, g, h). The nick in allele B: on the + strand (a, c, e, g), on the - strand (b, d, f, h).

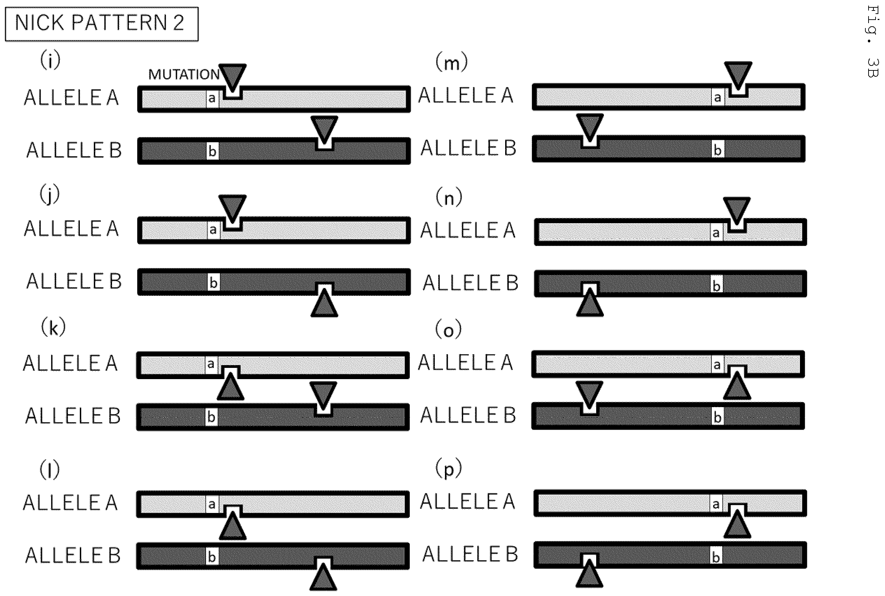

[Fig. 3B] Fig. 3B is a continuation of Fig. 3A. It differs from Fig. 3A in the position of the nick in allele A. The nick in allele A: on the + strand (i, j, m, n), on the - strand (k, l, o, p). The nick in allele B: on the + strand (i, k, m, o), on the - strand (j, l, n, p).

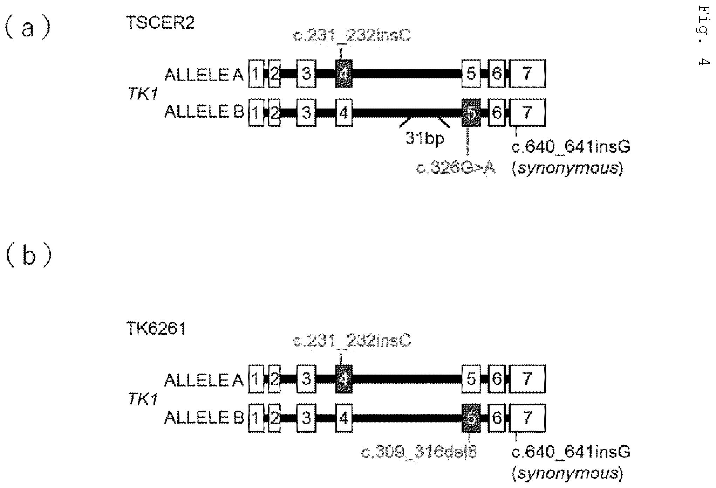

[Fig. 4] Fig. 4 provide schematic diagrams of the thymidine kinase gene in TSCER2 cells and TK6261 cells. (a) TSCER2 cells: include allele A, which has a loss-of-function frameshift due to a 1 bp insertion (c.231_232insC) in exon 4 of the thymidine kinase 1 gene, and allele B, which has a 31 bp insertion in intron 4, a loss-of-function point mutation (c.326G>A) on exon 5, and a 1 bp insertion (c.640_641insG) on exon 7 that does not affect function. (b) TK6261 cells: include allele A, which has a loss-of-function frameshift due to a 1 bp insertion (c.231_232insC) in exon 4 of the thymidine kinase 1 gene, and allele B, which has an 8 bp deletion (c.309_316del8) on exon 5 causing a loss-of-function frameshift and a 1 bp insertion (c.640_641insG) on exon 7 that does not affect function.

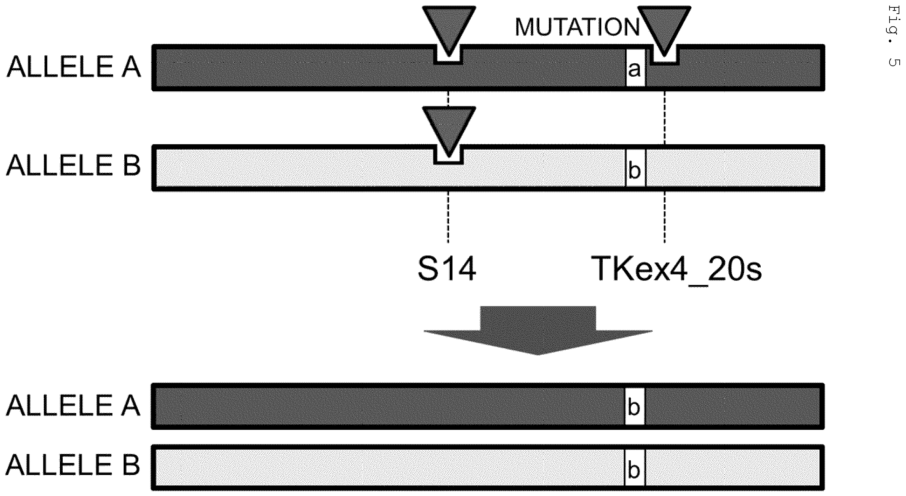

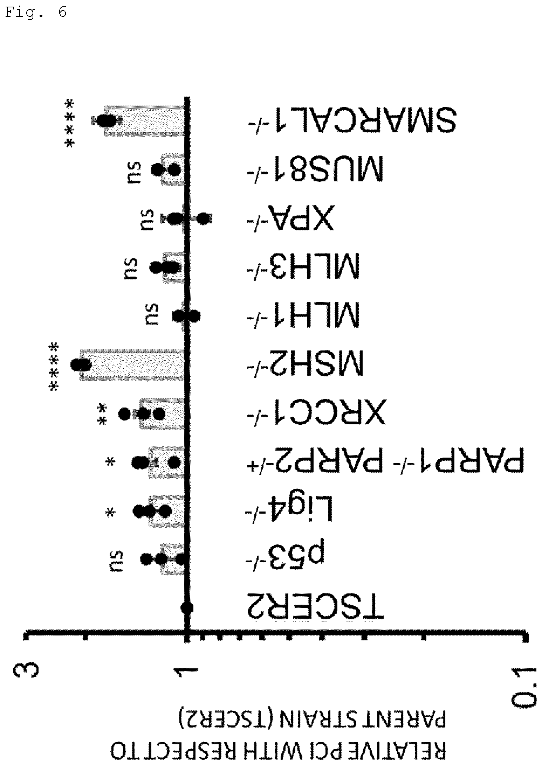

[Fig. 5] Fig. 5 is a schematic diagram of the genome editing method performed on the cells described in Fig. 6. By generating a nick specifically in allele A on the coding strand of the thymidine kinase gene with sgTKex4_20s and generating nicks on the coding strand of both allele A and allele B with sgS14, the mutation on allele A is substituted with the wild-type sequence on allele B.

[Fig. 6] Fig. 6 is a diagram showing the genome editing efficiency in knockout cells. The relative genome editing efficiency in each knockout cell was calculated when the genome editing efficiency in the parent strain TSCER2 was taken as 1. Genome editing efficiency was evaluated by the recovery of thymidine kinase. ****: $p < 0.0001$, **: $p < 0.01$, *: $p < 0.05$, ns: not significant.

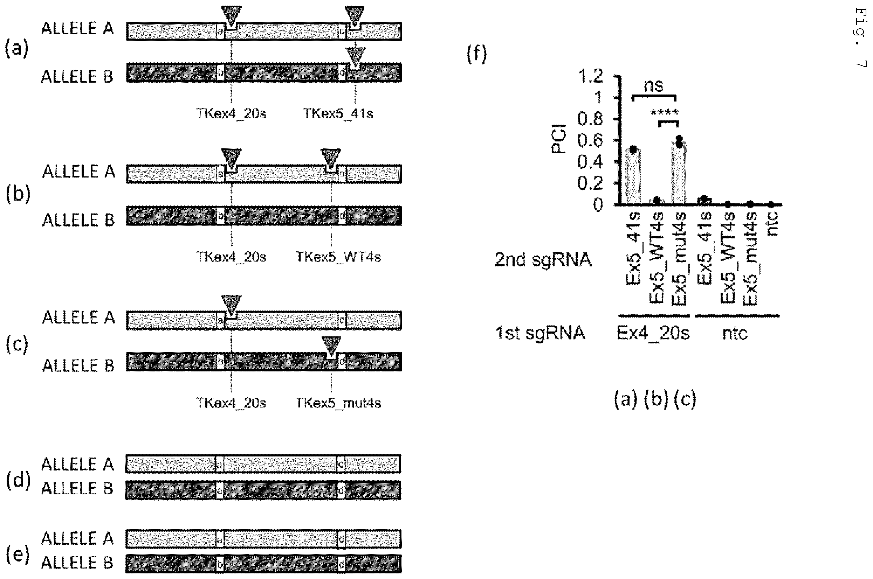

[Fig. 7] Fig. 7 is a diagram showing the relationship between the pattern of nicks introduced in each allele and the efficiency of genetic correction by homologous chromosome recombination. (a) to (c): Show the pattern of nicks in the thymidine kinase 1 gene on the chromosome used as the donor for homologous recombination. (d) and (e):

Show each allele in cells where the genetic defect was corrected. (f): A graph showing the proportion of cells in which the genetic defect was corrected and TK activity was restored. [a] and [c] in allele A show the mutant sequence. [b] and [d] in allele B show the wild-type sequence.

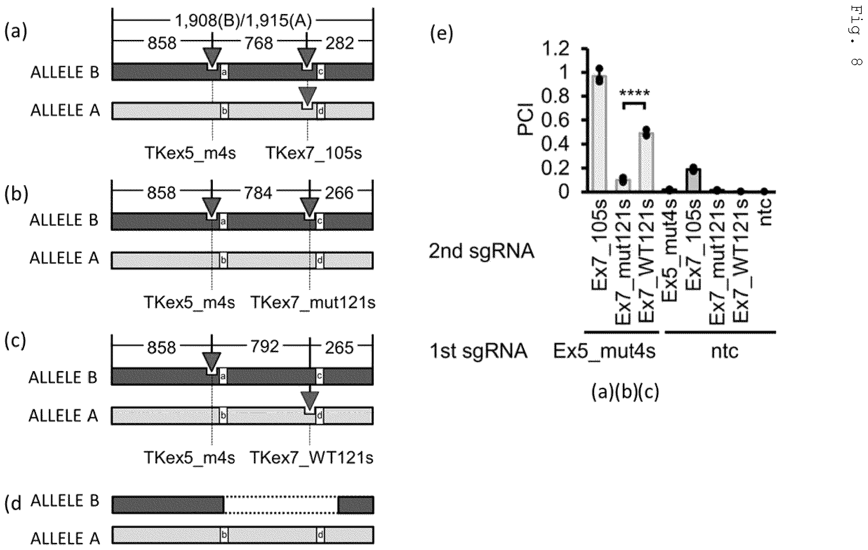

[Fig. 8] Fig. 8 is a diagram showing the relationship between the pattern of nicks introduced in each allele and the efficiency of genetic correction by homologous chromosome recombination. Experiments were performed at a different target site from Fig. 7. (a) to (c): Show the pattern of nicks in the thymidine kinase 1 gene on the chromosome used as the donor for homologous recombination. (d): Shows each allele in cells where the genetic defect was corrected. (e) A graph showing the proportion of cells in which the genetic defect was corrected and TK activity was restored. [a] in allele A shows the mutant sequence, and [c] shows the SNV sequence. [b] and [d] in allele B show the wild-type sequence.

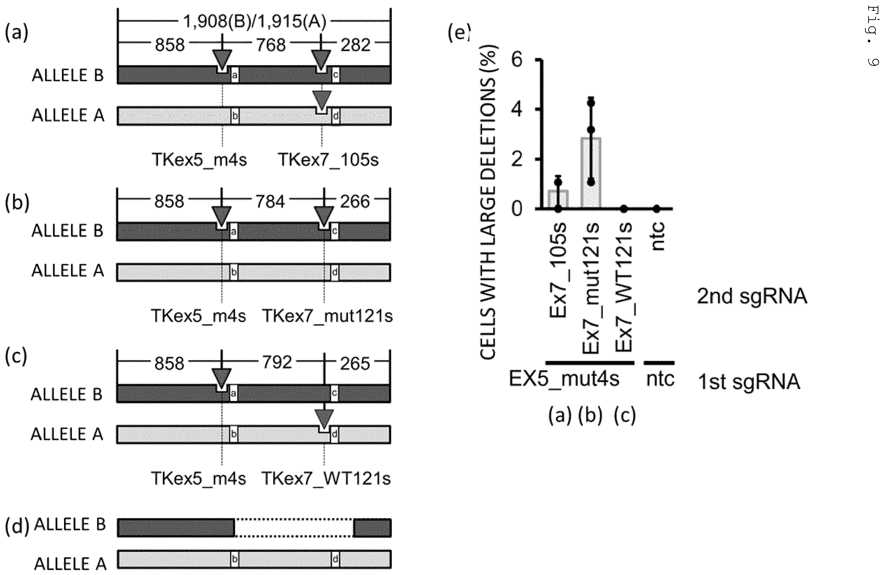

[Fig. 9] Fig. 9 is a diagram showing the relationship between the pattern of nicks introduced in each allele and the frequency of long-chain DNA deletions in homologous chromosomes. Cells subjected to genome editing treatment in the experiment in Fig. 8 were used. (a) to (c): Show the pattern of nicks in the thymidine kinase 1 gene on the chromosome used as the donor for homologous recombination. (d): Shows each allele in cells where long-chain DNA deletions occurred in allele B. (e): A graph showing the proportion of cells in which long-chain DNA deletions occurred in allele B. [a] in allele A shows the mutant sequence, and [c] shows the SNV sequence. [b] and [d] in allele B show the wild-type sequence.

[Description of Embodiments]

The method for producing a genome-edited cell in the present invention has a principle of unifying different bases between homologous chromosomes into any of the bases by utilizing homologous recombination between homologous chromosomes induced by single-strand breaks with site-specific nickase.

Specifically, the method includes introducing, into a cell having different bases between homologous chromosomes at a specific site of the homologous chromosomes, a combination of site-specific nickases that cleave a single strand in a neighboring DNA region of the specific site, inducing homologous recombination with one of the homologous chromosomes as a recipient and the other as a donor, and substituting the base of the recipient with the base of the donor at the specific site.

In the present invention, the "specific site on the homologous chromosomes" is the target site for genome editing on the homologous chromosomes. The "different bases between the homologous chromosomes" at this site may be one base or multiple bases (a base sequence). Moreover, it may be a mutation or a polymorphism. Examples of mutations include substitutions, deletions, insertions, or combinations thereof, and examples of polymorphism include single base polymorphism and microsatellite polymorphism. Note that the base may be present in the genetic coding region or in the genetic non-coding region.

In the present invention, among homologous chromosomes, chromosomes having mutations or polymorphisms at specific sites can be used as recipients or donors in homologous recombination. Specifically, by the genome editing in the present invention, the bases at specific sites of the two chromosomes constituting the homologous chromosomes can both be made into normal sequences, or both can be made into specific mutant sequences or polymorphic sequences. For example, in HLA, the heterozygous HLA can be made into homozygous HLA.

From the viewpoint of medical usefulness, a typical utilization mode of the present invention is to repair a mutation to a normal sequence in human cells in order to treat or prevent a disease caused by a heterozygous mutation. Here, "diseases caused by heterozygous mutations" include diseases directly caused by the heterozygous mutation (dominant genetic diseases) as well as diseases caused by a combination of two different mutations (compound heterozygotes) (recessive genetic diseases). Examples of target diseases include, but are not limited to, diseases with an autosomal dominant inheritance pattern that onset due to autosomal heterozygous mutations (for example, OAS1 abnormality in congenital immunodeficiency, severe congenital neutropenia caused by ELANE mutation, chronic mucocutaneous candidiasis, etc.), triplet repeat diseases with an autosomal dominant inheritance pattern (for example, Huntington's disease, spinocerebellar ataxia, myotonic dystrophy, etc.), genetic diseases with an autosomal recessive inheritance pattern (for example, ADA deficiency, etc.), and diseases that onset in females with an X-linked inheritance pattern (for example, hemophilia with Factor VIII and Factor IX deficiency, etc.).

The "site-specific nickase" used in the present invention is not limited as long as it can cleave a single strand of DNA in a site-specific manner on the genome, but is preferably a CRISPR-Cas system having a nickase-type Cas protein as a component. Cas proteins typically include a domain involved in cleavage of the target strand (RuvC domain) and a domain involved in cleavage of the non-target strand (HNH domain), whereas the nickase-type Cas protein typically loses its cleavage activity due to a mutation in either of these two domains. In the case of spCas9 protein (Cas9 protein derived from S. pyogenes), examples of such mutation include a mutation of the 10th amino acid (aspartic acid) from the N-terminus to alanine (D10A: mutation within the RuvC domain), a mutation of the 840th amino acid (histidine) from the N-terminus to alanine (H840A: mutation within the HNH domain), a mutation of the 863th amino acid (asparagine) from the N-terminus to alanine (N863A: mutation within the HNH domain), a mutation of the 762th amino acid (glutamic acid) from the N-terminus to alanine (E762A: mutation within the RuvCII domain), and a mutation of the 986th amino acid (aspartic acid) from the N-terminus to alanine (D986A: mutation within the RuvCIII domain). In addition, Cas9 proteins of various origins are known (for example, WO2014/131833), and their nickase types can be utilized. Note that amino acid sequences and base sequences of Cas9 proteins are registered in a public database, for example, GenBank (http://www.ncbi.nlm.nih.gov) (for example, accession number: Q99ZW2.1 and the like), and these can be used in the present invention.

Further, in the present invention, it is also possible to use Cas proteins other than Cas9, such as Cpf1 (Cas12a), Cas12b, CasX (Cas12e), and Cas14. Examples of mutations in the nickase-type Cpf1 proteins include, in AsCpf1 (Cas12), a mutation of the 1226th amino acid (arginine) from the N-terminus to alanine (R1226A: mutation within the Nuc domain). Amino acid sequences of Cpf1 are registered in a public database, for example, GenBank (http://www.ncbi.nlm.nih.gov) (for example, accession numbers: WP_021736722, WP_O35635841, and the like).

In the CRISPR-Cas system including a nickase-type Cas protein as a component, the nickase-type Cas protein binds to a guide RNA to form a complex, and is targeted to a target DNA sequence to cleave a single strand of DNA. In the CRISPR-Cas9 system, the guide RNA includes crRNA and tracrRNA, but in the CRISPR-Cpf1 system, for example, tracrRNA is unnecessary. The guide RNA in the CRISPR-Cas9 system may be a single-molecule guide RNA containing crRNA and tracrRNA, or a double-molecule guide RNA composed of a crRNA fragment and a tracrRNA fragment.

The crRNA contains a base sequence complementary to the target DNA sequence. The target DNA sequence is a base sequence composed of usually 12 to 50 bases, preferably 17 to 30 bases, and more preferably 17 to 25 bases, and is preferably selected from regions adjacent to the PAM (proto-spacer adjacent motif) sequence. Typically, site-specific cleavage of DNA takes place at positions determined by both the complementarity of base pairing between the crRNA and the target DNA sequence and the PAM present adjacent thereto.

In many CRISPR-Cas systems, crRNA also contains a base sequence on the 3'-side that can interact (hybridize) with the tracrRNA fragment. Meanwhile, tracrRNA contains a base sequence that can interact (hybridize) with a part of the base sequence of crRNA on the 5'-side. By the interaction of these base sequences, crRNA/tracrRNA (one molecule or two molecules) form a double-stranded RNA, and the formed double-stranded RNA interacts with the Cas protein.

PAM varies depending on the type and origin of Cas protein. Typical PAM sequences are, for example, "5'-NGG" for Cas9 protein (type II) derived from S. pyogenes, "5'-CCN" for Cas9 protein (type I-A1) derived from S. solfataricus, "5'-TCN" for Cas9 protein (type I-A2) derived from S. solfataricus, "5'-TTC" for Cas9 protein (type I-B) derived from H. walsbyi, "5'-AWG" for Cas9 protein (type I-E) derived from E. coli, "5'-CC" for Cas9 protein (type I-F) derived from E. coli, "5'-CC" for Cas9 protein (type I-F) derived from P. aeruginosa, "5'-NNAGAA" for Cas9 protein (type II-A) derived from S. Thermophilus, "5'-NGG" for Cas9 protein (type II-A) derived from S. agalactiae, "5'-NGRRT" or "5'-NGRRN" for Cas9 protein derived from S. aureus, "5'- NNNNGATT" for Cas9 protein derived from N. meningitidis, and "5'-NAAAAC" for Cas9 protein derived from T. denticola. In Cpf1, it is typically "5'-TTN" or "5'-TTTN." Note that it is also possible to modify PAM recognition by modifying the protein (for example, introducing a mutation) (Benjamin, P. et al., Nature 523, 481-485 (2015), and Hirano, S. et al., Molecular Cell 61, 886-894 (2016)).

In the present invention, a site-specific nickase other than CRISPR-Cas systems can also be used. Examples of such site-specific nickase include an artificial nuclease fused with an enzyme having nickase activity. Examples of artificial nucleases usable include TALE (transcription activator-like effector), ZF (zinc finger), and PPR (pentatricopeptide repeat). Examples of enzymes capable of exerting nickase activity by fusion with these artificial nucleases include TevI (Nat Commun. 2013; 4: 1762. doi: 10.1038/ncomms2782). These artificial nucleases are targeted to the target DNA sequence by a DNA-binding domain constructed by linking modules (peptides) that recognize specific bases (or specific base sequences), and cleave a single strand of DNA with a nickase fused to the DNA-binding domain. A suitable spacer peptide may be introduced between the DNA binding domain and the nickase in an artificial nuclease.

The first embodiment of the present invention uses a combination of site-specific nickases that causes single-strand breaks in the recipient chromosome at multiple sites in the neighboring DNA region of the specific site (different bases between homologous chromosomes), and causes a single-strand break in the donor chromosome at at least one of sites corresponding to the sites of single-strand breaks in the recipient chromosome. Examples of specific patterns of single-strand breaks are shown in Figs. 2A and 2B.

In the second embodiment of the present invention, a combination of site-specific nickases that causes a single-strand break at one site in the neighboring DNA region of the specific site on the chromosome of the recipient and causes a single-strand break at a different site from the site corresponding to the site where the single-strand break is caused on the chromosome of the recipient, at one site on the chromosome of the donor, is used. Examples of specific patterns of single-strand breaks are shown in Figs. 3A and 3B.

Here, the "neighboring DNA region" refers to a region within 100000 bases (for example, within 10000 bases, within 5000 bases, within 2000 bases, within 1000 bases, within 600 bases) from the specific site. The distance between the introduced nicks is typically 100 bases or more (for example, 200 bases or more, 300 bases or more, 500 bases or more, 700 bases or more, 1000 bases or more, 1500 bases or more). It is preferable that one of the nicks introduced into the chromosome is in the immediate vicinity of the specific site. Here, "immediate vicinity" typically means within about 100 bases, and more preferably within 50 bases (for example, within 40 bases, within 30 bases, within 20 bases, within 10 bases). The neighboring DNA region of one specific site may contain another specific site (different bases between homologous chromosomes). In the first embodiment, the "multiple sites in the neighboring DNA region" may be on the same DNA strand or on different DNA strands.

In the first embodiment, when simultaneously single-strand breaking corresponding sites on the chromosome of the donor and the chromosome of the recipient, site-specific nickases that bind to the target DNA sequence on the chromosome of the recipient may be designed to also bind to the corresponding DNA sequence on the chromosome of the donor. In this case, the target DNA sequence on the chromosome of the recipient and the corresponding DNA sequence on the chromosome of the donor are typically the same DNA sequence.

On the other hand, in the first and second embodiments, when causing a single-strand break only on the chromosome of the recipient at the corresponding sites on the chromosome of the donor and the chromosome of the recipient, some of the combinations of site-specific nickases that bind to the target DNA sequence on the chromosome of the recipient should be designed so that they do not bind to the corresponding DNA sequence on the chromosome of the donor. In this case, the target DNA sequence on the chromosome of the recipient and the corresponding DNA sequence on the chromosome of the donor are typically different DNA sequences. For example, if the target DNA sequence of the site-specific nickase is set to include bases that differ between homologous chromosomes, the target DNA sequence on the chromosome of the recipient and the corresponding DNA sequence on the chromosome of the donor will be different DNA sequences.

In the second embodiment, when causing a single-strand break only on the chromosome of the donor at the corresponding sites on the chromosome of the donor and the chromosome of the recipient, some of the combinations of site-specific nickases that bind to the target DNA sequence on the chromosome of the donor should be designed so that they do not bind to the corresponding DNA sequence on the chromosome of the recipient. In this case, the target DNA sequence on the chromosome of the donor and the corresponding DNA sequence on the chromosome of the recipient are typically different DNA sequences.

In the embodiment where a single-strand break is caused only on one chromosome at the corresponding sites on the chromosome of the donor and the chromosome of the recipient, when using the CRISPR-Cas system as the site-specific nickase, the guide RNA should be designed so that it has binding specificity to the target DNA sequence on one chromosome. Also, in the case where the site-specific nickase is an artificial nuclease fused with a nickase activity-bearing enzyme, the DNA binding domain may be designed to have binding specificity to the target DNA sequence on one chromosome. In this embodiment, due to the design of the site-specific nickase, the site of single-strand break is usually within about 100 bases, and more preferably within 50 bases (for example, within 40 bases, 30 bases, 20 bases, or 10 bases), from the specific site (different bases between homologous chromosomes).

In the first embodiment of the present invention, when the multiple site single-strand breaks on the chromosome of the recipient are caused on different DNA strands, double-strand breaks may take place if the single-strand breaks are too close together. Therefore, the distance between single-strand breaks on different DNA strands is usually 100 bases or more, and preferably 200 bases or more, and is usually within 2000 bases, preferably within 1000 bases, and further preferably within 500 bases.

In the present invention, the combination of site-specific nickases described above is introduced into cells. In the case of a CRISPR-Cas system, the "site-specific nickases" introduced into the cells may be, for example, in the form of a combination of a guide RNA and a Cas protein, in the form of a combination of a guide RNA and a messenger RNA translated into Cas protein, or a combination of vectors expressing them, for example. The guide RNA may be modified (such as chemical modification) to suppress degradation. In the case of an artificial nuclease fused with an enzyme having nickase activity, for example, they may be in the form of a protein, a messenger RNA translated into the protein, or in the form of a vector expressing the protein.

When employing the form of an expression vector, it includes one or more regulatory elements that are operatively bound to the DNA to be expressed. Here, "operatively bound" means that the DNA is operatively bound to the regulatory elements. Examples of the "regulatory elements" include promoters, enhancers, internal ribosome entry sites (IRES), and other expression control elements (such as transcription termination signals, for example polyadenylation signals and polyU sequences). Depending on the intended purpose, the regulatory elements may, for example, direct the constitutive expression of DNA in a variety of host cells, or may direct DNA expression only in specific cells, tissues, or organs. Further, they may direct the expression of DNA only at a specific time, or may direct the expression of artificially inducible DNA. Examples of promoters include polIII promoters (such as U6 and H1 promoters), polII promoters (such as retrovirus Rous sarcoma virus (RSV) LTR promoter, cytomegalovirus (CMV) promoter, SV40 promoter, dihydrofolate reductase promoter, β-actin promoter, phosphoglycerate kinase (PGK) promoter, and EF1α promoter), polI promoters, and combinations thereof. Those skilled in the art can select an appropriate expression vector according to the type and the like of cells for introduction.

The "cell" that is the target of genome editing in the present invention is not limited as long as it has a homologous chromosome, and various types of eukaryotic cells can be targeted.

Examples of "eukaryotic cells" include animal cells, plant cells, algae cells, and fungal cells. In addition to mammalian cells, examples of the animal cells include cells of fish, birds, reptiles, amphibians, and insects.

Examples of the "animal cells" include cells constituting an individual animal, cells constituting an organ-tissue extracted from an animal, cultured cells derived from an animal tissue, and the like. Specific examples include embryonic cells of embryos at various stages (such as 1-cell stage embryos, 2-cell stage embryos, 4-cell stage embryos, 8-cell stage embryos, 16-cell stage embryos, and morula stage embryos); stem cells such as induced pluripotent stem (iPS) cells, embryonic stem (ES) cells, and hematopoietic stem cells; and somatic cells such as fibroblasts, hematopoietic cells, neurons, muscle cells, osteocytes, hepatocytes, pancreatic cells, brain cells, and kidney cells. For the preparation of a genome-edited animal, a fertilized oocyte, that is, a fertilized egg, can be used. Particularly preferably, the fertilized egg is that of a pronuclear stage embryo. For the oocyte before fertilization, the cryopreserved one can be thawed and used.

In the present invention, "mammal" is a concept including human and non-human mammals. Examples of non-human mammals include even-toed ungulates such as cows, wild boars, pigs, sheep, and goats, odd-toed ungulates such as horses, rodents such as mice, rats, guinea pigs, hamsters, and squirrels, lagomorphs such as rabbits, and carnivores such as dogs, cats, and ferrets. The above-mentioned non-human mammal may be a domestic animal or a companion animal (pet animal), or may be a wild animal.

Examples of "plant cells" include cells of cereals, oil crops, fodder crops, fruits, and vegetables. Examples of the "plant cells" include cells constituting an individual plant, cells constituting an organ or tissue separated from a plant, cultured cells derived from a plant tissue, and the like. Examples of plant organs and tissues include leaves, stems, shoot apices (growth points), roots, tubers, root tubers, seeds, callus, and the like. Examples of plants include rice, corn, bananas, peanuts, sunflowers, tomatoes, oilseed rape, tobacco, wheat, barley, potatoes, soybeans, cotton, carnations, and the like.

In the first embodiment of the method of the present invention, it is preferable to use cells in which the function of a gene selected from the group consisting of Lig4 gene, PARP1 gene, XRCC1 gene, MSH2 gene, and SMARCAL1 gene is suppressed. The typical base sequence of the human-derived Lig4 gene and the amino acid sequence of the protein encoded by the gene are disclosed in databases (accession numbers: NM_002312, NM_206937, NM_001098268, NP_002303, NP_996820, NP_001091738), the base sequence of the human-derived PARP1 gene and the amino acid sequence of the protein encoded by the gene are disclosed in databases (accession numbers: NM_001618, NP_001609), the base sequence of the human-derived XRCC1 gene and the amino acid sequence of the protein encoded by the gene are disclosed in databases (accession numbers: NM_006297, NP_006288), the base sequence of the human-derived MSH2 gene and the amino acid sequence of the protein encoded by the gene are disclosed in databases (accession numbers: NM_000251 or NM_001258281, NP_000242, NP_001245210), and the base sequence of the human-derived SMARCAL1 gene and the amino acid sequence of the protein encoded by the gene are disclosed in databases (accession numbers: NM_014140, NM_001127207, NP_054859, NP_001120679), respectively.

"Suppression of gene function" in the present invention may have a mechanism that suppresses the activity of the translation product (protein) of the gene or suppresses the expression of the gene. Suppression of the activity of the translation product (protein) of the gene can be caused by introducing mutations (such as deletion, substitution, and insertion) into the gene or by inhibitor treatment, for example.

Introduction of mutations into genes can be caused using genome editing systems, for example. Examples of genome editing systems include site-specific artificial nucleases, such as CRISPR-Cas (e.g., CRISPR-Cas9) systems, TALENs, and ZFNs. The action of genome editing systems cleaves genes at target sites, and mutations (base deletions, insertions, etc.) are introduced into genes through DNA repair mechanisms in cells. When making the gene deficient by introducing mutations, the deficiency may be a deficiency of the entire gene or a partial deficiency.

Examples of inhibitor treatment include treatment with the Lig4 inhibitor SCR7, treatment with PARP inhibitors such as olaparib, veliparib, niraparib, or talazoparib, but are not limited to these.

"Suppression of gene function" can also be achieved by suppressing the expression of the gene. Suppression of gene expression may be suppression of translation or suppression of transcription of the gene. Suppression of translation of genes can be caused using double-stranded RNA (dsRNA) that binds to the transcription product of the gene, for example. Examples of double-stranded RNA include siRNA, shRNA (short hairpin RNA), miRNA, etc. Suppression of transcription of genes can be caused by introducing mutations into transcriptional regulatory regions of genes, for example. The above-mentioned genome editing systems targeting the transcriptional regulatory regions can be used to introduce mutations. Whether or not the expression of genes has been suppressed can be evaluated by immunological assay methods using antibodies that bind to translation products at the translational level, and by methods such as RT-PCR and Northern blotting at the transcriptional level.

As for molecules that suppress the function of genes, these molecules themselves may be introduced into cells, or in the case where these molecules are nucleic acids, DNA encoding the molecules (typically vectors into which the DNA is inserted) may be introduced into cells for intracellular expression.

The introduction of site-specific nickases and molecules that inhibit the function of the gene into cells can be performed by a known method such as electroporation, microinjection, DEAE-dextran method, lipofection method, nanoparticle-mediated transfection method, and virus-mediated nucleic acid delivery method.

After introduction into cells, the combination of site-specific nickases causes single-strand breaks in the chromosomes of the recipient and donor. This induces homologous recombination between homologous chromosomes, and the target base of the recipient is efficiently and specifically substituted with the corresponding base of the donor.

The present invention also provides a kit for use in the method of the present invention, comprising a combination of the above site-specific nickases. The kit may further comprise one or more additional reagents, and examples of additional reagents include, but are not limited to, dilution buffers, reconstitution solutions, wash buffers, nucleic acid introduction reagents, protein introduction reagents, and control reagents (e.g., control guide RNAs). The kit may include instructions for use to practice the method of the present invention.

[Examples]

Hereinafter, the present invention is described in more detail based on Examples, but the present invention is not limited to the following Examples.

[Materials and Methods]

A. Materials

(1) TSCER2 cells (Fig. 4a)

TSCER2 cells, which are lymphoblastoid cells derived from TK6 cells, include allele A, which has a loss-of-function frameshift due to a 1 bp insertion (c.231_232insC) in exon 4 of the thymidine kinase 1 gene, and allele B, which has a 31 bp insertion in intron 4, a loss-of-function point mutation (c.326G>A) on exon 5, and a 1 bp insertion (c.640_641insG) on exon 7 that does not affect function.

(2) Knockout cells derived from TSCER2 cells

These are cells in which the following genes were knocked out in TSCER2 cells by gene targeting. p53$^{-/-}$, Lig4$^{-/-}$, PARP1$^{-/-}$PARP2$^{-/+}$, XRCC1$^{-/-}$, MSH2$^{-/-}$, MLH1$^{-/-}$, MLH3$^{-/-}$, XPA$^{-/-}$, MUS81$^{-/-}$, or SMARCAL1$^{-/-}$. The knockout cells for each gene are registered in the TK6 Mutants Consortium (https://www.nihs.go.jp/dgm/tk6.html).

(3) TK6261 cells (Fig. 4b)

TK6261 cells, which are lymphoblastoid cells derived from TK6 cells, include allele A, which has a loss-of-function frameshift due to a 1 bp insertion (c.231_232insC) in exon 4 of the thymidine kinase 1 gene, and allele B, which has an 8 bp deletion (c.309_316del8) on exon 5 causing a loss-of-function frameshift and a 1 bp insertion (c.640_641insG) on exon 7 that does not affect function.

(4) TK6261 EGFP cells

This is a strain in which the EGFP gene is knocked into the Rosa26 region of TK6261 cells.

(5) Cas9D10A nickase mRNA

CleanCap Cas9 Nickase mRNA (5moU)-(L-7207) (TriLink BioTechnologies) was used at 1.0 mg/mL.

(6) Target sequences of sgRNA (PAM sequences in parentheses)

sgTKex4_20s: CGTCTCGGAGCAGGCAGGCG(GGG) / SEQ ID NO: 1
sgS14: TCCCACCGAAGGCCACACGC(CGG) / SEQ ID NO: 2
sgS11: TCAATCATATCACTCTTAGC(TGG) / SEQ ID NO: 3
sgS12: GGAGCTGTCCATGAGACCCA(GGG) / SEQ ID NO: 4

sgTKex5_mut4s: CTCGCAGAACTCCAGGGAAC(TGG) / SEQ ID NO: 5
sgTKex5_WT4s: ACTCCACGATGTCAGGGAAC(TGG) / SEQ ID NO: 6
sgTKex5_41s: AGCCACAATTACGGTCTTCC(CGG) / SEQ ID NO: 7
sgTKex7_105s: CCCCTGGCTTTCCTGGCACT(GGG) / SEQ ID NO: 8
sgTKex7_WT121s: CTGGCAGCCACGGCTTCCCC(TGG) / SEQ ID NO: 9.
TKex7mt_121s: TGGCAGCCACGGCTTCCCCC(TGG) / SEQ ID NO: 10

(7) Electroporator

Invitrogen Neon Transfection System, Neon Transfection System 10 μL Kit (Thermo Fisher Scientific).

(8) Basal medium

Medium in which 5% horse serum (Invitrogen) and 200 ug/ml Sodium Pyruvate (Nacalai) are added to RPMI 1640 medium (Nacalai).

(9) CHAT medium

Medium in which 10 μM 2-deoxycytidine [Sigma], 200 μM hypoxanthine [Sigma], 100 nM aminopterin [Sigma], and 17.5 μM thymidine [Sigma] are added to the basal medium.

(10) PCR primers

For PCR in which the target sequences of sgRNA "TKex5_mut4s," sgRNA "TKex7_105s," and sgRNA "TKex7_mut121s" are included in the PCR product (full length about 1.9 kbp), the primer set L-TKex57 was used.

L-TKex57 Forward: AAGCCCTCACGTCTCAATAACC / SEQ ID NO: 11
L-TKex57 Reverse: GCTTTAAGCAGACCAGTGGGTA / SEQ ID NO: 12

B. Methods

A mixture of 0.9 μL of 1.0 mg/mL Cas9D10A mRNA, 0.45 μL each of two types of sgRNA at 100 nmol/mL, and 2.2 μL of Neon Transfection System R buffer was prepared for a total of 4 μL, and 10 μL of R buffer containing 70 × $10^4$ cells was mixed with it. Electroporation was performed on 10 μL of this mixture using the Neon Transfection System under the conditions of Voltage 1500 V, Width 10 ms, Pulse 3 pulses.

[Example]

A. Verification of the effect of knockout of specific genes on genome editing efficiency

Electroporation of two types of sgRNA (sgTKex4_20s and sgS14) and Cas9D10A mRNA was performed on TSCER2 cells, TSCER2-derived p53$^{-/-}$, Lig4$^{-/-}$, PARP1$^{-/-}$PARP2$^{-/+}$, XRCC1$^{-/-}$, MSH2$^{-/-}$, MLH1$^{-/-}$, MLH3$^{-/-}$, XPA$^{-/-}$, MUS81$^{-/-}$, SMARCAL1$^{-/-}$ cells, and TK6261 EGFP cells (Fig. 5). Four days after electroporation, TK6261 EGFP cells as standard cells and TSCER2 cells or TSCER2-derived knockout cells as test cells were mixed at a cell count ratio of 1:1, and some were cultured for 8 days in basal medium, while others were cultured for 4 days in CHAT medium and then for 4 days in basal medium. After culturing, the proportions of EGFP positive cells and negative cells were measured by flow cytometry. It has already been demonstrated that in cells that proliferate in CHAT medium after electroporation of sgTKex4_20s and sgS14 with Cas9D10A mRNA, exon 4 of the TK1 gene becomes the homozygous wild type. The proliferation-ability in CHAT medium index (PCI) was calculated from the ratio of EGFP positive cell rate after selection in CHAT medium to EGFP positive cell rate in basal medium (Table 1).

$$PCI = x1 \cdot y2 \ / \ (x2 \cdot y1)$$

[Table 1]

|  | Standard Cell | Test Cell |
|---|---|---|
|  | EGFP Positive (%) | EGFP Negative (%) |
| Culture in Basal Medium | x1 | x2 |
| Culture in CHAT Medium | y1 | y2 |

Fig. 6 shows the relative PCI in each knockout cell when the PCI in TSCER2 is taken as 1. An increase in genome editing efficiency was observed by knockout of Lig4, PARP1, MSH2, and SMARCAL1.

The detailed method for calculating PCI is as follows.

Suppose that a% of the standard cells (EGFP positive cells) occupying x1% of the cells in the basal medium have succeeded in genetic correction, and b% of the test cells (EGFP negative) occupying x2% of the cells in the basal medium have succeeded in genetic correction. Then, the following equation holds.

$$x1 \cdot a \; : \; x2 \cdot b \; = \; y1 \; : \; y2$$

$$x2 \cdot y1 \cdot b \; = \; x1 \cdot y2 \cdot a$$

PCI is the ratio of the genetic correction rate in test cells to that in standard cells (b/a), and the following equation holds.

$$PCI \; = \; b/a \; = \; x1 \cdot y2 \; / \; (x2 \cdot y1)$$

B. Influence of the pattern of single-strand breaks in homologous chromosomes on the efficiency and specificity of genome editing

(1) Relationship between the pattern of nicks introduced in each allele and the efficiency of genetic correction by homologous chromosome recombination

(i) Experiment 1

An sgRNA (sgTKex5_mut4s) that specifically targets allele B at the 8 bp deletion in exon 5 of TK6261 cells, an sgRNA (sgTKex5_WT4s) that specifically targets allele A using the same PAM sequence as this sgRNA, and an sgRNA (sgTKex5_41s) that targets both allele A and allele B were each combined with sgTKex4_20s and introduced into TK6261 cells by electroporation method together with Cas9D10A nickase mRNA.

For TK6261 EGFP cells, a combination of sgTKex4_20s and sgS11 was introduced by electroporation method together with Cas9D10A nickase mRNA.

Four days after electroporation, TK6261 EGFP cells and TK6261 cells were mixed at a cell count ratio of 1:1, and some were cultured for 8 days in basal medium, while others were cultured for 4 days in CHAT medium and then for 4 days in basal medium. After culturing, the proportions of EGFP positive cells and negative cells were measured by flow cytometry. The proliferation in CHAT medium index (PCI) was calculated from the ratio of EGFP positive cell rate after selection in CHAT medium to EGFP positive cell rate in basal medium. This allows calculation of the proportion of cells in which the genetic defect was corrected and TK activity was restored as shown in (d) or (e) .

When nicks were generated at one site each in both homologous chromosomes (sgTKex4_20s + sgTKex5_mut4s: (c)), TK activity was restored much more efficiently than when tandem nicks were generated only in allele A (sgTKex4_20s + sgTKex5_WT4s: (b)) (Fig. 7). Also, when tandem nicks were generated in allele A and one nick was generated in allele B (sgTKex4_20s + sgTKex5_41s: (a)), TK activity was restored with equivalent efficiency to when nicks were generated at one site each in both homologous chromosomes (c) (Fig. 7).

(ii) Experiment 2

An sgRNA (sgTKex7_WT121s) that specifically targets allele A, which has the wild-type sequence in exon 7, or an sgRNA (sgTKex7_105s) that targets both allele A and allele B in exon 7 was combined with an sgRNA (sgTKex5_mut4s) that specifically targets allele B, which has the mutant sequence in exon 5, and introduced into TK6261 cells by electroporation together with Cas9D10A nickase mRNA.

For TK6261 EGFP cells, a combination of sgTKex4_20s and sgS12 was introduced by electroporation together with Cas9D10A nickase mRNA.

Four days after electroporation, TK6261 EGFP cells and TK6261 cells were mixed at a cell count ratio of 1:1, and some were cultured for 8 days in basal medium, while others were cultured for 4 days in CHAT medium and then for 4 days in normal medium. After culturing, the proportions of EGFP positive cells and negative cells were measured by flow cytometry. The proliferation in CHAT medium index (PCI) was calculated from the ratio of EGFP positive cell rate after selection in CHAT medium to EGFP positive cell rate in basal medium. This allows calculation of the proportion of cells in which the genetic defect was corrected and TK activity was restored as shown in (d).

When one nick each was generated in allele A and allele B (sgTKex5_mut4s + sgTKex7_WT121s: (c)), genome editing was performed significantly more efficiently than when tandem nicks were generated only in allele B (sgTKex5_mut4s + sgTKex7_mut121s: (b)), and about half the genome editing efficiency was maintained compared to when tandem nicks were generated in allele B and one nick was generated in allele A (sgTKex5_mut4s + sgTKex7_105s: (a)) (Fig. 8).

(2) Relationship between the pattern of nicks introduced in each allele and the frequency of long-chain DNA deletions in homologous chromosomes

For the cells subjected to 8 days of culturing after the introduction of each genome editing system in the above (1) Experiment 2, sorting was performed using FACS Aria III (BD) so that there was 1 cell/well in a 96-well plate with 200 $\mu$L of basal medium dispensed per well. After about one week of culturing, 94 clones derived from a single cell that proliferated were collected, and genomic DNA was extracted using an Easy DNA Extraction Kit Version 2 (Kaneka).

Using the DNA extracted from single cell-derived clones as a template, PCR was performed using KOD FX Neo (TOYOBO). The PCR products were electrophoresed in a 0.9% TAE gel, and after staining the gel with Ultra Safe Dye, they were visualized under blue LED light and photographed with a digital camera. When tandem nicks were generated in allele B and one nick was generated in allele A (sgTKex5_mut4s + sgTKex7_105s: the method of the filed patent: (a)), large deletions of 500 bp or more (Fig. (d)) occurred at 0.71 + 0.61%, but when one nick each was generated in allele A and allele B (sgTKex5_mut4s + sgTKex7_WT121s: (c)), it was 0% (did not occur at all) (Fig. 9).

[Industrial Applicability]

As described above, according to the present invention, it is possible to unify different bases between homologous chromosomes to either base by utilizing homologous recombination between homologous chromosomes induced by single-strand cleavage by a site-specific nickase. Since the present invention does not use exogenous donor DNA, it can greatly contribute to gene therapy for diseases caused by heterozygous mutations, especially in terms of its high safety.

[Sequence Listing Free Text]

SEQ ID NOs: 1-10: sgRNA target sequences
SEQ ID NOs: 11-12: primer sequences

**Claims**

1.  A method for producing genome-edited cells, comprising:

    introducing a combination of site-specific nickases that causes single-strand breaks in a neighboring DNA region of a specific site in homologous chromosomes having different bases between the homologous chromosomes at the specific site, inducing homologous recombination using one of the homologous chromosomes as a recipient and the other as a donor, and substituting a base of the recipient with a base of the donor at the specific site, wherein
    the cells have suppressed function of a gene selected from the group consisting of Lig4 gene, PARP1 gene, XRCC1 gene, MSH2 gene, and SMARCAL1 gene, and
    the combination of site-specific nickases causes single-strand breaks at multiple sites in the neighboring DNA region of the specific site on the chromosome of the recipient and causes a single-strand break at one site corresponding to the site where the single-strand breaks are caused on the chromosome of the recipient, on the chromosome of the donor.

2. The method according to claim 1, wherein the base of the recipient to be substituted is a mutant base and the base of the donor is a normal base.

3. The method according to claim 1 or 2, wherein each of the site-specific nickases is a CRISPR-Cas system.

4. A kit for use in the method according to any one of claims 1 to 3, comprising:

a combination of site-specific nickases that causes single-strand breaks in a neighboring DNA region of a specific site of homologous chromosomes in cells having different bases between the homologous chromosomes at the specific site, wherein
the cells have suppressed function of a gene selected from the group consisting of Lig4 gene, PARP1 gene, XRCC1 gene, MSH2 gene, and SMARCAL1 gene, and
the combination of site-specific nickases causes single-strand breaks at multiple sites in the neighboring DNA region of the specific site on the chromosome of the recipient and causes a single-strand break at one site corresponding to the site where the single-strand breaks are caused on the chromosome of the recipient, on the chromosome of the donor.

5. A method for producing genome-edited cells, comprising:

introducing a combination of site-specific nickases that causes single-strand breaks in a neighboring DNA region of a specific site in homologous chromosomes having different bases between the homologous chromosomes at the specific site, inducing homologous recombination using one of the homologous chromosomes as a recipient and the other as a donor, and substituting a base of the recipient with a base of the donor at the specific site, wherein
the combination of site-specific nickases causes a single-strand break at one site in the neighboring DNA region of the specific site on the chromosome of the recipient and causes a single-strand break at a different site from the site corresponding to the site where the single-strand break is caused on the chromosome of the recipient, at one site on the chromosome of the donor.

6. The method according to claim 5, wherein the base of the recipient to be substituted is a mutant base and the base of the donor is a normal base.

7. The method according to claim 5 or 6, wherein each of the site-specific nickases is a CRISPR-Cas system.

8. A kit for use in the method according to any one of claims 5 to 7, comprising:

a combination of site-specific nickases that causes single-strand breaks in a neighboring DNA region of a specific site of homologous chromosomes in cells having different bases between the homologous chromosomes at the specific site, wherein
the combination of site-specific nickases causes a single-strand break at one site in the neighboring DNA region of the specific site on the chromosome of the recipient and causes a single-strand break at a different site from the site corresponding to the site where the single-strand break is caused on the chromosome of the recipient, at one site on the chromosome of the donor.

Fig. 1

EP 4 455 282 A1

Fig. 2A

Fig. 2B

NICK PATTERN 2

Fig. 3B

Fig. 4

( a )

TSCER2

c.231_232insC

*TK1*

ALLELE A | 1 | 2 | 3 | 4 | 5 | 6 | 7 |

ALLELE B | 1 | 2 | 3 | 4 | 5 | 6 | 7 |

31bp

c.326G>A

c.640_641insG
(*synonymous*)

( b )

TK6261

c.231_232insC

*TK1*

ALLELE A | 1 | 2 | 3 | 4 | 5 | 6 | 7 |

ALLELE B | 1 | 2 | 3 | 4 | 5 | 6 | 7 |

c.309_316del8

c.640_641insG
(*synonymous*)

Fig. 5

EP 4 455 282 A1

Fig. 6

21

Fig. 7

Fig. 8

Fig. 9

(e)

CELLS WITH LARGE DELETIONS (%)

6  4  2  0

ntc — ntc — 1st sgRNA

Ex7_WT121s
Ex7_mut121s
Ex7_105s — (a) (b) (c) — 2nd sgRNA

EX5_mut4s

(a)

858  768  282
1,908(B)/1,915(A)

ALLELE B
ALLELE A

TKex5_m4s  TKex7_105s

(b)

858  784  266

ALLELE B
ALLELE A

TKex5_m4s  TKex7_mut121s

(c)

858  792  265

ALLELE B
ALLELE A

TKex5_m4s  TKex7_WT121s

(d) ALLELE B
ALLELE A

24

## INTERNATIONAL SEARCH REPORT

International application No.

**PCT/JP2022/046917**

### A. CLASSIFICATION OF SUBJECT MATTER

*C12N 15/09*(2006.01)i; *C12N 5/10*(2006.01)i; *C12N 9/16*(2006.01)i
FI:  C12N15/09 110; C12N9/16 Z; C12N5/10 ZNA

According to International Patent Classification (IPC) or to both national classification and IPC

### B. FIELDS SEARCHED

Minimum documentation searched (classification system followed by classification symbols)

C12N15/09; C12N5/10; C12N9/16

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN), JSTPlus/JMEDPlus/JST7580 (JDreamIII), GenBank/EMBL/DDBJ/GeneSeq, PubMed, Japio-GPG/FX

### C. DOCUMENTS CONSIDERED TO BE RELEVANT

| Category* | Citation of document, with indication, where appropriate, of the relevant passages | Relevant to claim No. |
|---|---|---|
| Y | WO 2020/100361 A1 (OSAKA UNIVERSITY) 22 May 2020 (2020-05-22) claims 1-4 | 5-8 |
| A | claims 1-4 | 1-4 |
| Y | JP 2018-011525 A (OSAKA UNIVERSITY) 25 January 2018 (2018-01-25) claim 1, example 3, fig. 3 | 5-8 |
| A | claim 1, example 3, fig. 3 | 1-4 |
| A | JP 2019-521139 A (VERTEX PHARMACEUTICALS INCORPORATED) 25 July 2019 (2019-07-25) entire text, all drawings | 1-8 |
| A | NAKAJIMA, K., et al. Precise and efficient nucleotide substitution near genomic nick via noncanonical homology-directed repair. Genomic Research. 2018, vol. 28, pp. 223-230 entire text, all drawings | 1-8 |

☐ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **03 March 2023** | **14 March 2023** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

**INTERNATIONAL SEARCH REPORT**

International application No.

**PCT/JP2022/046917**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "In the form of an Annex C/ST.25 text file" above should be understood as "in ST.26 format".

Form PCT/ISA/210 (continuation of first sheet) (January 2015)

INTERNATIONAL SEARCH REPORT
Information on patent family members

International application No.

**PCT/JP2022/046917**

| Patent document cited in search report | | | Publication date (day/month/year) | Patent family member(s) | | | Publication date (day/month/year) |
|---|---|---|---|---|---|---|---|
| WO | 2020/100361 | A1 | 22 May 2020 | US | 2021/0324420 | A1 | |
| | | | | claims 1-4 | | | |
| | | | | EP | 3882347 | A1 | |
| JP | 2018-011525 | A | 25 January 2018 | (Family: none) | | | |
| JP | 2019-521139 | A | 25 July 2019 | WO | 2018/013840 | A1 | |
| | | | | entire text, all drawings | | | |
| | | | | US | 2019/0225990 | A1 | |
| | | | | EP | 3484870 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018011525 A
- WO 2020100361 A
- WO 2014131833 A

**Non-patent literature cited in the description**

- **NAKAJIMA K et al.** *Genome Res.,* 2018, vol. 28, 223-230
- **BENJAMIN, P. et al.** *Nature,* 2015, vol. 523, 481-485
- **HIRANO, S. et al.** *Molecular Cell,* 2016, vol. 61, 886-894
- *Nat Commun.,* 2013, vol. 4, 1762